# FASCICULE DE BREVET EUROPEEN

(11) **EP 1 007 047 B1**
(45) Date de publication et mention de la délivrance du brevet: **14.12.2005**
(21) Numéro de dépôt: 98921544.7
(22) Date de dépôt: 16.04.1998
(51) Int. Cl.: A61K 31/505, A61P 29/00

(54) **UTILISATION DE LA MIZOLASTINE POUR LA PREPARATION D'UN MEDICAMENT DESTINE AU TRAITEMENT DE L'INFLAMMATION**
VERWENDUNG VON MIZOLASTINE ZUR HERSTELLUNG EINES ARZNEIMITTELS ZUR BEHANDLUNG VON ENTZÜNDUNGEN
USE OF MIZOLASTINE FOR PREPARING A MEDICINE FOR TREATING INFLAMMATION

(30) Priorité: 17.04.1997 FR 9704801
(43) Date de publication de la demande: 14.06.2000
(73) Titulaire: Sanofi-Aventis, 75013 Paris (FR)
(72) Inventeur: ANGEL, Itzchak, Nes-Ziyyona (IL); ARBILLA, Sonia, F-75007 Paris (FR); EVEN, Luc, F-75015 Paris (FR); GOLDHILL, Jon, F-75007 Paris (FR); PICHAT, Philippe, F-91400 Orsay (FR); ROOME, Nigel, F-27120 Saint Aquilin de Pacy (FR)
(74) Mandataire: Thouret-Lemaitre, Elisabeth
(86) Numéro de dépôt international: PCT/FR1998/000765
(87) Numéro de publication internationale: WO 1998/047511

(56) Documents cités:
- EP-A- 0 217 700
- LEVRIER ET AL.: "anti-anaphylactic activity of the novel selective histamine H1 receptor antagonist mizolastine in the rodent" ARZNEIMITTELFORSCHUNG, vol. 45, no. 5, 1995, pages 559-568, XP002049056
- LEYNADIER ET AL.: "efficacy and safety of mizolastine in seasonal allergic rhinitis" ANNALS OF ALLERGY, ASTHMA AND IMMUNOLOGY, vol. 76, no. 2, 1996, pages 163-168, XP002049057
- PINQUIER ET AL.: "effect of mizolastine on experimental inflammation in human skin" CLINICAL PHARMACOLOGY AND THERAPEUTICS, vol. 57, no. 2, 1995, page 169 XP002049058
- ANGEL ET AL.: "powerful antihistamine properties of mizolastine in cutaneous oedema in the dog" ALLERGY, vol. 51, no. 31, 1996, page 171 XP002049059

## Description

La présente invention a pour objet l'utilisation de la mizolastine, 2-[[1-[1-[(4-fluorophényl)méthyl]-1*H*-benzimidazol-2-yl]pipéridin-4-yl]méthylamino]pyrimidin-4-ol ou 2-[[1-[1-[(4-fluorophényl)méthyl]-1*H*-benzimidazol-2-yl]pipéridin-4-yl]méthylamino]pyrimidin-4(1H)-one, et de ses sels pharmaceutiquement acceptables, pour la préparation d'un médicament destiné au traitement des maladies inflammatoires ou composantes inflammatoires de certaines autres maladies, liées à la voie de la 5-lipoxygénase.

La mizolastine et ses sels pharmaceutiquement acceptables sont décrits dans le brevet européen EP 0 217 700. La mizolastine est connue comme antagoniste des récepteurs de l'histamine (H1) et est utilisée dans le traitement de manifestations allergiques diverses.

Certains documents traitant de la mizolastine évoquent son effet dans les inflammations allergiques reliées à la libération d'histamine : Levrier et al., "anti-anaphylactic activity of the novel selective histamine Hl receptor antagonist mizolastine in the rodent", *Arzneimittelforschung,* **45**(5), 559-568 (1995); Leynadier et al., "efficacy and safety of mizolastine in seasonal allergic rhinitis", *Annals of Allergy*, astma and immunology, **76**(2), 163-168 (1996); Pinquier et al., "effect of mizolastine on experimental inflammation in human skin", *Clinical pharmacolgy and* therapeutics, **57**(2), 169 (1995) ; Angel et al., "powerful antihistamine properties of mizolastine in cutaneous oedema in the dog", *Allergy,* 51(31), 171 (1996).

Il s'agit dans les cas décrits dans ces documents des réactions inflammatoires induites indirectement par l'histamine (His) tel que l'a décrit Anderson, R. et al. dans "The in vitro effects of histamine and metiamide on neutrophil motility and their relationship to intracellular cyclic nuclotide levels", J. Immunol.*,* 118, 1690-1657 (1977).

Effectivement, l'histamine (His) n'est pas stricto *sensu* un médiateur de l'inflammation mais participe à l'altération physiologique durant les processus inflammatoires installés. His est principalement libérée en réponse à une stimulation par un antigène, à certains facteurs d'inflammation cellulaires ou à des stimuli physiques. Le rôle direct de His a été décrit et caractérisé dans les réactions allergiques mais seulement dans les phases aiguës de l'allergie. His n'est pas considérée comme directement impliquée dans l'initiation de l'inflammation dans les phases retardées de l'allergie (Tannenbaum et al. *J. Immunol.**125**,* 325).

En effet, His est une amine stockée essentiellement dans les cellules mastocytaires et basophiles. Elle est par elle-même uniquement capable de produire des effets intradermiques de vasodilatation, d'accroissement de perméabilité vasculaire locale ou de douleur, soit seulement 3 des 5 signes cardinaux de l'inflammation. Ainsi, concernant les cellules inflammatoires principales, c'est à dire les neutrophiles (cellules myéloïdes), l'histamine par elle-même, ne modifie pas le chimiotactisme de base de ces cellules. De plus, His n'affecte que le chimiotactisme cellulaire déjà stimulé qui est associé à l'augmentation intracellulaire du taux d'adénosine monophosphate 3'5' cyclique (AMPc), principalement au travers de l'activité agoniste des sous-types de récepteurs à l'histamine H₂.

Contrairement à His, les leukotriènes jouent un rôle clé dans les réponses inflammatoires et sont impliqués dans la genèse de diverses pathologies inflammatoires.

Plus précisément, les leukotriènes dérivent en fait d'un précurseur commun, le leukotriène A4 (LTA4). Celui-ci n'est formé qu'après une étape intermédiaire dans laquelle l'acide hydroxyperoxyéicosatrénique (5-HPETE) est synthétisé par action de la 5-lipoxygénase(5-LO) sur l'acide arachidonique (AA).

Ainsi, la réduction de la voie de la 5-LO est une action potentielle d'inhibition de la production des leukotriènes impliqués dans les processus inflammatoires (Bell et al., *Journal of Lipid Mediators,* **6**, 259-264, 1993; R.M. McMillan et E. R. H. Wlaker, *Trends Pharmacol. Sci.,* **13**, 323-330, 1992).

Il faut préciser également que, plus en amont, les sites primaires d'activation de la biosynthèse de l'acide arachidonique suite à un stimulus inflammatoire sont limités aux cellules myéloides (neutrophiles, éosinophiles,..), (conduisant aux métabolites de la 5-LO) et sont sous la dépendance d'un mécanisme de translocation du calcium du cytosol vers la membrane cellulaire. Ce mécanisme de transduction du message inflammatoire est, dès lors, tout à fait différent de celui connu pour His qui lui-même est relié au système de transduction nucléotidique (AMPcyclique / GMPcyclique).

Le demanderesse a trouvé que la mizolastine inhibe la 5-LO. La présente invention a donc pour objet l'utilisation de la mizolastine et de ses sels pharmaceutiquement acceptables, pour la préparation d'un médicament destiné au traitement des maladies inflammatoires ou composantes inflammatoires de certaines autres maladies, liées à la voie de la 5-LO.

La mizolastine a été testée *in vitro* sur la 5-LO et *in vivo* dans des modèles d'inflammation chez le rat.

L'activité *in vitro* de la mizolastine a été étudiée sur la 5-LO chez le rat et chez l'homme.

### Exemple 1 : Inhibition de la 5-LO chez le rat

Les essais sont réalisés selon le protocole décrit par R.W. Egan et P.H. Gale dans *J*. *Biol. Chem.,* **260**, 1154-1159, 1985, avec de la 5-LO issue d'une préparation de cellules basophiles leucémiques de rat (RBL-1). La mizolastine est incubée avec l'enzyme pendant 5 minutes à température ambiante et la réaction est initiée par addition d'acide linoléique. Après 8 minutes d'incubation à température ambiante, la réaction est terminée par addition de soude et l'absorption est lue à 234 nm pour déterminer le taux de 5-HETE.

L'activité de la mizolastine, exprimée en concentrations inhibitrices (CI₅₀) est de 4,6 *µ*M.

### Exemple 2 : Inhibition de la 5-LO chez l'homme

La mizolastine a été testée sur la 5-LO isolée à partir de cellules HL60 commerciales. La mizolastine est incubée pendant 10 minutes à température ambiante en présence d'acide arachidoniqe (0,4 *µ*M) et de 5-LO. L'expérience est réalisée simultanément en présence d'acide nordihydrogualarétique comme produit de référence. La quantité de 5-HETE formée est déterminée par RIA (Coffrey et al., *J*. *Biol. Chem.,* 267-270, 1992).

L'activité de la mizolastine, exprimée en concentrations inhibitrices (CI₅₀) est de 16 *µ*M.

L'activité *in vivo* de la mizolastine a été étudiée dans un modèle d'inflammation de la patte de rat et dans un modèle d'inflammation de la colite de rat.

### Exemple 3 : Activité in vivo de la mizolastine dans un modèle d'inflammation de la patte de rat

### a) inflammation par injection d'AA

L'oedème inflammatoire de la patte de rat induit par l'injection d'AA est réalisé et évalué selon la méthode de Di Martino et al. (*Agents and actions,* **21**, 303-305, 1987) à l'exception près que la concentration d'AA utilisé est de 0,3% (v/v).

La mizolastine est donnée oralement 2 heures avant l'injection d'AA. Une solution d'AA, préparée extemporanément dans une solution tampon (0,1 ml ; carbonate 0,2 M ; pH = 11,2) est injectée dans la partie subplantaire de la patte postérieure droite de rat.

Le volume de la réaction inflammatoire est mesuré par plethysmographie après 1, 2, 3 et 4 heures.

La mizolastine à la dose de 0,3 mg/kg, confère une inhibition durable de 55%, par rapport au contrôle, de l'inflammation induite après 4 heures.

### Exemple 4 : Activité in vivo de la mizolastine dans un modèle d'inflammation de colite expérimentale de rat

La mizolastine a été étudiée dans un modèle de recto-colite de rat induite par l'acide trinitrobenzène sulfonique (TNBS) selon la méthode décrite par Morris et al. (Gastroenterology, 96, 795-803, 1989).

La mizolastine est donnée oralement (3 mg/kg) une heure avant et un jour après l'instillation de TNBS (40 mg/kg dans l'éthanol à 50%) dans la partie distale du colon. Après 3 jours, la mesure de l'index macroscopique des dommages, de l'oedème/hypertrophie (poids de tissus) et de l'activité myeloperoxydase comme mesure de l'infiltration des neutrophiles est effectuée. La mizolastine confère une réduction significative des dommages, de l'oedème/hypertrophie (poids de tissus) et de l'activité myeloperoxydase respectivement de 67%, 57% et 66% à 3 mg/kg.

Les résultats de ces essais montrent que la mizolastine et ses sels pharmaceutiquement acceptables peuvent être utilisés pour la fabrication de médicaments destinés au traitement des maladies inflammatoires ou composantes inflammatoires de certaines autres maladies, liées à la voie de la 5-LO, parmi lesquelles on peut citer : les réactions allergiques, les rhinites, l'asthme, la détresse respiratoire aiguë de l'adulte, l'arthrite rhumatoïde, la fibrose cystique le psoriasis, le syndrome de l'intestin inflammatoire, la gastrite, la maladie de Crohn, l'iléocolite, l'entérocolite, la recto-colite hémorragique (colite ulcéreuse) et la colite inflammatoire.

A cet effet, la mizolastine et ses sels peuvent être présentés sous toutes formes pharmaceutiques adaptées à l'administration orale ou topique, en association avec des excipients appropriés pour permettre une administration journalière de 10 à 210 mg en principe actif.

## Revendications

1. Utilisation de la mizolastine et de ses sels pour la fabrication d'un médicament destiné au traitement de l'arthrite rhumatoïde, la fibrose cystique, le psoriasis, le syndrome de l'intestin inflammatoire, la gastrite, la maladie de Crohn, l'iléocolite, l'entérocolite, de la rectocolite hémorragique et la colite inflammatoire.

2. Utilisation de la mizolastine pour inhiber une 5-lypoxygénase isolée.

## Patentansprüche

1. Verwendung von Mizolastin und seiner Salze für die Herstellung eines Arzneimittels, das zur Behandlung von rheumatoider Arthritis, zystischer Fibrose, Schuppenflechte, Syndrom des entzündlich inflammatorisch veränderten Intestinaltrakts, Gastritis, Crohnscher Krankheit, Ileitis, Enterokolitis, hämorrhagischer Rektokolitits und inflammatorischer Kolitis bestimmt ist.

2. Verwendung von Mizolastin zur Hemmung einer isolierten 5-Lypoxygenase.

## Claims

1. Use of mizolastine and salts thereof for the manufacture of a medicament intended for treating rheumatoid arthritis, cystic fibrosis, psoriasis, inflammatory intestine syndrome, gastritis, Crohn's disease, ileocolitis, enterocolitis, haemorrhagic rectocolitis and inflammatory colitis.

2. Use of mizolastine to inhibit an isolated 5-lipoxygenase.
